# EUROPEAN PATENT SPECIFICATION

(11) **EP 0 753 009 B1**
(45) Date of publication and mention of the grant of the patent: **16.05.2001**
(21) Application number: 95913269.7
(22) Date of filing: 30.03.1995
(51) Int. Cl.: C07K 14/445, A61K 39/015, C12N 5/08, C12N 15/30

(54) **MALARIA PEPTIDES**
MALARIAPEPTIDE
PEPTIDES DE LA MALARIA

(30) Priority: 31.03.1994 GB 9406492
(43) Date of publication of application: 15.01.1997
(73) Proprietor: Oxxon Pharmaccines Limited, Oxford, OX1 3AW (GB)
(72) Inventor: HILL, Adrian, Vivian, Sinton, Oxford 0X3 9DL (GB); AIDOO, Michael, Oxford 0X3 9DU (GB); ALLSOPP, Catherine, Elizabeth, Margaret, Oxford 0X3 7EH (GB); LALVANI, Ajit, Oxford 0X2 6JA (GB); PLEBANSKI, Magdalena, Oxford 0X3 7EH (GB); WHITTLE, Hilton, Carter, P.O. Box 273 Fajara Near Banjul (GM)
(74) Representative: Maschio, Antonio
(86) International application number: GB9500737
(87) International publication number: WO9526982

(56) References cited:
- WO-A-90/00402
- WO-A-90/01496
- WO-A-93/20103
- NATURE, vol. 360, 3 December 1992 LONDON, GB, pages 434-439, A.V.S. HILL ET AL. 'MOLECULAR ANALYSIS OF THE ASSOCIATION OF HLA-B53 AND RESISTANCE TO SEVERE MALARIA.' cited in the application
- NATURE, vol. 335, 1 September 1988 LONDON, pages 79-82, K.J.H. ROBSON 'A HIGHLY CONSERVED AMINO-ACID SEQUENCE IN THROMBOSPONDIN, PROPERDIN AND IN PROTEINS FROM SPOROZOITES AND BLOOD STAGES OF A HUMAN MALARIA PARASITE.' cited in the application
- MOLECULAR AND BIOCHEMICAL PARASITOLOGY, vol. 48, no. 2, 1991 AMSTERDAM, NL, pages 223-226, J. ZHU ET AL. 'STRUCTURE OF PLASMODIUM FALCIPARUM LIVER STAGE ANTIGEN-1.' cited in the application
- SCIENCE, vol. 225, no. 4662, 10 August 1984 LANCASTER, PA, US, pages 593-599, J.B. DAME ET AL. 'STRUCTURE OF THE GENE ENCODINGTHE IMMUNODOMINANT SURFACE ANTIGEN ON THE SPOROZOITE OF THE HUMAN MALARIA PARASITE PLASMODIUM FALCIPARUM.' cited in the application
- THE LANCET, vol. 345, 22 April 1995 LONDON, GB, pages 1003-1007, M. AIDOO ET AL. 'IDENTIFICATION OF CONSERVED ANTIGENIC COMPONENTS FOR A CYTOTOXIC T LYMPHOCYTE-INDUCING VACCINE AGAINST MALARIA.'

## Description

### INTRODUCTION

The identification of peptide epitopes for HLA class I molecules is of importance for several areas of biomedical science. Firstly, such epitopes are the central components of vaccines designed to provide immunity mediated by cytotoxic T lymphocytes (CTL) to infectious microorganisms. This is a consequence of the method whereby cytotoxic T lymphocytes function: they recognise a peptide epitope of 8-11 amino acids in length presented by an HLA class I molecule. Secondly, there is increasing interest in the possibility that neoplastic tumours might be ameliorated or cured by inducing, either in vivo or in vitro, CTL that can recognize tumour-specific peptides on HLA class I molecules. Thirdly, the identification of such epitopes recognised in autoimmune disorders would provide insights into pathogenesis and suggest new specific methods of treating these disorders.

This invention deals firstly with the identification of peptides from the malaria parasite *Plasmodium falciparum* which we identify as epitopes or potential epitopes for particular HLA class I molecules. A variety of evidence (cited in references 1 and 16) suggests that CTL play a role in immunity to this parasitic infection and disease by acting against the liver-stage of infection. Thus these epitopes will be of value for inclusion in vaccines designed to provide immunity to *Plasmodium falciparum*. Furthermore, this work identifies for the first time CTL epitopes in the *P. falciparum* antigen, thrombospondin-related anonymous protein (TRAP²), and thus identifies TRAP, and / or peptides from TRAP, as a useful component of a CTL-inducing vaccine against *P*. *falciparum* malaria.

This invention deals secondly with a novel method of identifying cytotoxic T lymphocyte epitopes and of producing CTL lines and clones by the use of a new method of inducing CTL responses *in vitro.*

### THE INVENTION

In one aspect the invention provides the 9-mer peptides set out in the table below, being either epitopes or potential epitopes for HLA-A2 class I molecules, conservative variants thereof, and longer peptides containing these sequences which are sub-units of the indicated antigens. It is envisaged that these peptides are generally 8 to 100 amino acid residues in length, and that the sequence shown in the table is the main functional epitope present. No per se claim is made to the antigen as a whole, nor to any fragment which constitutes the larger part of the antigen.

Two or more of these peptides may be joined together in sequence. The peptide may have an N- or C- terminus carrying a lipid tail, a modification known to enhance CTL induction *in vivo*.

Also included are oligonucleotides which code for the stated peptides. The term oligonucleotide is here used to encompass nucleic acid chains of about 24 to about 300 nucleotide residues.

Also included are vaccines comprising peptides or oligonucleotides as defined, for immunisation against malaria.

### SECTION A

### Identification of P. falciparum peptides and epitopes

We have recently described a novel approach to identifying CTL epitopes and potential CTL epitopes in *P*. *falciparum*¹. This consisted, in brief, of 1) determining a motif for peptides bound to a particular HLA class I molecules, 2) synthesizing peptides from *P*. *falciparum* antigens congruent with this motif, 3) testing whether these peptides bind to that HLA class I allele using a binding assay known as a HLA assembly assay, and 4) testing whether lymphocytes from individuals exposed to malaria could recognise these peptides as epitopes after suitable in vitro restimulation and culture. In that work we identified peptide epitopes and potential peptide epitopes for two HLA class I molecules, HLA-B53 and HLA-B35. Here we extend that work to a further HLA class I molecule: HLA-A2. 11 peptides are identified that are shown either to be epitopes or potential epitopes for this HLA class I molecule.

### Detailed description

### Motifs for eluted peptides.

The peptide binding motifs of HLA-A2 have been described. Strong preferences were found at positions 2 and 9 of bound peptides: for leucine, isoleucine and methionine at position 2, and for valine, leucine and isoleucine at position 9^{3,4}.

### Peptide Synthesis and Binding Assays

As described previously for identification of HLA-B35 and HLA-B53 epitopes¹, peptides were synthesised to correspond to these four motifs from the primary amino acid sequences of four *P. falciparum* pre-erythrocytic antigens: CSP, TRAP, LSA-1 and SHEBA. Over one hundred peptides were synthesized. Binding assays⁷ were performed on selected peptides to determine whether they bound to HLA-A2. using the untransfected T2 cell line.

### Cytotoxic T Lymphocyte assays

Peptides shown to bind to a particular HLA class I allele were tested for CTL recognition in assays using lymphocytes from malaria-exposed Gambians, as described previously¹. A minority of peptides, synthesized to correspond to a peptides binding motif were not tested in the relevant assembly assay but tested only for CTL recognition. Cells from 82 adult Gambians and 53 Gambian children, all exposed to malaria, were used in the course of these studies. The children and adults were HLA typed using cellular or molecular techniques as described previously¹. Peptides were incubated with cells either singly, or in pools as described¹ at concentrations of 10-100*µ*M. Peptides could either be left with the cells for the duration of restimulation (as reported¹) or washed off after an hour. Cells were cultured for 1-3 weeks before a standard CTL chromium release assay¹ was performed using HLA matched or autologous B cell line targets pre-pulsed with the peptide to be tested. Peptides were tested either singly or in pools for CTL recognition. 10% or greater was regarded as a significant level of specific lysis.

### Peptides Identified: CTL Epitopes.

The sequences of all peptides referred to are shown in the table.

Two Gambian individuals showed positive CTL responses to HLA-A2 peptides. One individual (Z62) recognised a pool of three TRAP peptides, tr26, tr29 and tr39. Insufficient cells were available to determine which of these three peptides was the epitope. However a second adult (Z60) also recognised this pool of three peptides and subsequently showed significant lysis of tr39- but not tr26-, nor tr29-pulsed target cells. Hence tr39 is an HLA-A2-restricted epitope. Both individuals were of the HLA-A*0201 subtype of HLA-A2. All three of these peptides bound to HLA-A2 in the assembly assay.

Further studies consisting of cytotoxic Tlymphocyte assays using lymphocytes from malaria-exposed Gambians have confirmed that the following peptides listed in the table are cytotoxic Tlymphocyte epitopes. For individuals with HLA-A2, CTL from at least one individual have been found to recognise the peptides tr26, tr29, cp36, cp37, cp38 and cp39. These epitopes for HLA-A2 are in addition to tr39 identified previously.

### Further peptides.

In addition to these epitopes identified above further peptides were identified as "potential epitopes" as follows. The designation "potential epitope" is justified as follows. All of these peptides are from antigens shown to be targets of CTL recognition. They are furthermore capable of binding with high affinity to the specified HLA class I molecule. Although they have not been shown to be recognised by CTL grown from malaria exposed individuals their ability to bind to the relevant class I molecule indicates that they are likely to be presented on the surface of malaria-infected hepatocytes *in vivo*. Hence, even if detectable levels of CTL to these peptides are not found in malaria-exposed individuals, induction of CTL to these peptides may be a useful means of immunizing against *P*. *falciparum* malaria.

The following peptides were shown to bind to HLA-A2 in the HLA assembly assay and are therefore potential CTL epitopes: ls10, ls11, ls19, ls23. The peptide cp36 was also capable of inducing a primary CTL response in malaria-unexposed individuals (see below).

Further evidence for the potential usefulness of the peptides tr43, cp36 and variants of cp36 (cp37-39) in a malaria vaccine is presented below where we show that CTL may be generated *in vitro* against these peptides from malaria un-exposed individuals.

This work identifies TRAP as a *P. falciparum* antigen which induces cytotoxic T lymphocyte responses in individuals exposed to endemic malaria. TRAP is expressed on sporozoites⁹ as well as blood-stage malaria parasite and will therefore be present in the infected liver cell. We show here that TRAP contains CTL epitopes for the very common HLA class I antigen, HLA-A2 and therefore the induction of CTL to TRAP may be an important requirement of an effective CTL-inducing vaccine against *P*. *falciparum*.

As there is evidence that better CTL responses may be induced *in vivo* using epitopes or epitopes with a limited amount of flanking sequence than by using the whole antigen, the epitopes used here may be particularly valuable for the induction of CTL responses *in vivo*¹⁵.

This specification identifies for the first time the existence of CTL responses to the antigen TRAP in humans exposed to *P*. *falciparum* parasites. It is known from studies of rodent malaria and indirectly from studies of human *P*. *falciparum* malaria¹⁶ that CTL are likely to play a protective role but the target antigens of these CTL have been unclear. By identifying TRAP as a target of CTL responses in humans we identify it as a favourable antigen for inclusion in a vaccine designed to induce protective CTL responses. Moreover, we show here that TRAP contains conserved CTL epitopes for the very common class I antigen, HLA-A2, which is the most prevalent HLA-A or -B molecule in Caucasians, making TRAP of particular importance for immunization through CTL of Caucasian populations.

There are several means by which the CTL epitopes identified here may be used to stimulate an immune response *in vivo* in humans. Either the peptides, or longer peptides containing them, can be used alone or with an adjuvant, such as incomplete Freund's adjuvant¹⁷ or QS-21¹⁸ or NAGO¹⁹ or AF²⁰, or as peptides with a lipid-tail added²¹, a means that has been shown to enhance CTL induction *in vivo*. Alternatively, the epitopes can be delivered by recombining nucleotides encoding them into a gene coding for a particle such as a recombinant Ty-virus-like-particle²² or a recombinant hepatitis B virus antigen particle²³. Alternatively, nucleotides encoding these epitopes can be incorporated into a recombinant virus such as a vaccinia virus or an attenuated vaccinia virus²⁴. Another means is to generate a recombinant bacterium such as a recombinant *Salmonella* containing nucleotides encoding these epitopes²⁵. Another means is to incorporate nucleotides encoding the epitopes identified into an expression vector, such as a DNA vaccine²⁶, that can express these epitopes after immunization. Finally, ribonucleotides coding for these epitopes can be used as an RNA-based vaccine²⁷ to express these epitopes *invivo*.

### SECTION B

### A Method of Inducing Primary CTL Responses In Vitro

Although secondary (or recall) CTL responses to a variety of infectious micro-organisms can now be detected (e.g. as described for malaria above), CTL cannot be grown in this way from individuals unexposed to antigen or microorganism (ref.1 and unpublished data). We describe here a novel method of growing "primary" CTL i.e. from previously unprimed individuals. This method can be employed for generating cell lines and clones which may be useful in various ways: to identify potential epitopes amongst a pool of peptides which bind to an HLA class I molecule; to identify peptides presented by HLA molecules on the surface of a cell using a CTL assay; for *in vivo* therapeutic use for the treatment of infectious or neoplastic disease. We demonstrate this method by describing the generation of CTL lines and clones to two peptides from *P*. *falciparum* from the lymphocytes of three individuals who have not been exposed to or infected by this parasite.

The method described here for inducing primary CTL responses *in vitro* may be particularly useful in cancer immunotherapy. Studies in mice have demonstrated the potential of therapy with *ex vivo* cultured CTL²⁸, and human tumor-specific CTL have been identified in the peripheral blood or tumor-infiltrating lymphocytes from patients with melanoma and renal cell carcinoma^{29,30}. Additionally, induction of CTL against viral antigen epitopes *in vitro* may be useful in the therapy of viral infections such as HIV³¹.

### Detailed description

Peripheral blood mononuclear cells from two HLA-A2 individuals never exposed to malaria were separated on Ficoll-hypaque and prepulsed for 2 hours with 20-100*µ*M peptide cp36. The cells were then washed once and 5 million cells were incubated in a 2 ml well (in a standard humidified incubator with 5% CO₂) in Ó-MEM (minimal essential medium, GIBCO, UK) with autologous heat-inactivated human serum and 2*µ*g per ml of keyhole limpet haemocyanin (KLH, Calbiochem, California, USA). The addition of the latter was based on our previous showing that this preferentially stimulates the CD45RA⁺ (naive) subset of CD4 T lymphocytes^{10,11}. This CD4 T lymphocyte subset has been shown previously to promote CD8 T cell activity¹².

After 72 hours interleukin-2 (Cetus, California, USA) was added at a concentration of 10 units per ml and the cells were cultured for a further 4 days. Then 5000 of the cells were restimulated in 150*µ*l of Ó-MEM with 10% autologous human serum with 100,000 irradiated autologous peripheral blood lymphocytes, that had been pre-pulsed for one hour in 20*µ*M of the same peptide used for the first stimulation and then washed once. On the following day 3 units of IL-2 was added to the well and the incubation continued for 8 further days. Then standard CTL assays were performed at an effector to target ratio of 5-10:1. Peptide specific lysis in excess of 10% was observed in from 5 - 15% of wells. This CTL activity was peptide-specific and dependent on CD8⁺ lymphocytes. In the case of cp36 the CTL responses were shown to be HLA-A2.1 restricted.

**TABLE**

| label | Sequence | | | | | | | | | | Position |
|---|---|---|---|---|---|---|---|---|---|---|---|
| HLA-A2 | 1 | 2 | 3 | 4 | 5 | 6 | 7 | 8 | 9 | 10 | |
| tr26 * | H | L | G | N | V | K | Y | L | V | | 3 |
| tr29 | L | L | M | D | C | S | G | S | I | | 51 |
| tr39 * | G | I | A | G | G | L | A | L | L | | 500 |
| Is10 | I | L | Y | I | S | F | Y | F | I | | 4 |
| Is11 | Y | I | S | F | Y | F | I | L | V | | 6 |
| Is19 | G | I | Y | K | E | L | E | D | L | | 1801 |
| Is23 | H | I | F | D | G | D | N | E | I | | 1883 |
| cp36 * | Y | L | K | T | I | Q | N | S | L | | 334 |
| cp37 * | Y | L | Q | K | I | Q | N | S | L | | 334 |
| cp38 * | Y | L | Q | K | I | K | N | S | L | | 334 |
| cp39 * | Y | L | N | K | I | Q | N | S | L | | 334 |

| | | | | | | | | | | | |
|---|---|---|---|---|---|---|---|---|---|---|---|
| * Peptide identified as an epitope for a secondary cytotoxic T lymphocyte response. | | | | | | | | | | | |

Table: Peptides from three *Plasmodium falciparum* antigens, circumsporozoite protein (cp), thrombospondin-related anonymous protein (tr) and liver-stage antigen-1 (ls), that are here identified as CTL epitopes or as potential CTL epitopes for particular HLA class I molecules. Epitopes are shown in bold type. The position of the first amino acid of the peptide in the published amino acid sequence (CSP - ref. 13; LSA-1 - ref. 14; TRAP - ref. 2) is shown. The standard one letter amino acid code is used.

### References

1. Hill, A.V.S. et al. *Nature* **360**, 434-439 (1992).
2. Robson, K.J.H. et al. *Nature* **335**, 79-82 (1988).
3. Falk, K., Rotzscke, O., Stevanovic, S., Jung, G. & Rammensee, H. *Nature* **351**, 290- 296 (1991).
4. Hunt, D.F. et al. *Science* **255**, 1261-6 (1992)
5. Huczko, E.L. et al. *J*. *Immunol*. **151**, 2572-2587 (1993)
6. Sutton, J. et al. *Eur*. *J*. *Immunol*. **23,** 447-453 (1993).
7. Elvin, J. et al. *J*. *Immunol*. *Methods* **158,** 161-171 (1992)
8. Doolan, D.A., Saul, A. & Good, M.F. *Infection and Immunity* **60**, 675-682 (1992)
9. Cowan, G., Krishna, S., Crisanti, A. & Robson, K.J.H. *Lancet* **339**, 1412-1413
10. Plebanski, M., Saunders, M., Burtles, S.S., Crowe, S. & Hooper, D.C. *Immunology* **75**: 86-92 (1992).
11. Plebanski, M. & Burtles, S.S. *J. Immunol*. *Methods* In Press (1994).
12. Morimoto, C. et al. *Eur*. *J. Immunol*. **16,** 198-204 (1986).
13. Dame, J.B. et al. *Science* **225**, 593-599 (1984).
14. Zhu, J. & Hollingdale, M. *Mol*. *Biochem*. *Parasitol*. **48**, 223-226 (1991).
15. Lawson, C. M. *et al*. *J*. *Virol*. **68:** 3505-11 (1994).
16. Lalvani, A, *et al*. *Research in Immunology* **145:** 461-468 (1994).
17. Kast, W.M. *et al. Proc Natl Acad Sci USA* **88:** 2282-2287 (1991).
18. Newman, M.J. *et al*. *J*. *Immunol*. **148:** 2357-64 (1992).
19. Zheng, B. *et al*. *Science* **256:** 1560-1563 (1992).
20. Raychaudhuri *et al*. *Proc Natl Acad Sci USA* **89:** 8308-8312 (1992).
21. Deres, K. *et al*. *Nature* **342:** 561-3 (1989).
22. Layton, G.T. *et al*. *J*. *Immunol*. **151**: 1097-1107 (1993).
23. Tindle, R.W. *et al*. *Virology* **200:** 547-557 (1994).
24. Cox, W.I. *et al*. *Virology* **195:** 845-850 (1993).
25. Chatfield, S.N. *et al*. *Vaccine* **10:** 53-60 (1992).
26. Ulmer, J.B. *et al*. *Science* **259:** 1745-49 (1993).
27. Martinon, F. *et al*. *Eur*. *J*. *Immunol*. **23:** 1719-22 (1993).
28. Greenberg, P.D. *Adv*. *Immunol*. **49:** 281 (1991).
29. Cerottini, J.C. *et al*. *Ann*. *Oncol*. **3:** 11 (1992).
30. Koo, A.S. *et al*. *J*. *Immunotherapy* **10:** 347 (1991).
31. Riddell, S.R. & Greenberg, P.D. *Curr*. *Top*. *Microbiol*. *Immunol*. **189:** 9-34 (1994).

## Claims

1. The peptides, being either epitopes or potential epitopes for HLA (human leucocyte antigen)-A2 class I molecules, conservative variants thereof, and longer peptides containing these sequences which are sub-units of the indicated antigens:
| label | Sequence | | | | | | | | | | Position |
|---|---|---|---|---|---|---|---|---|---|---|---|
| HLA-A2 | 1 | 2 | 3 | 4 | 5 | 6 | 7 | 8 | 9 | 10 | |
| tr26 | H | L | G | N | V | K | Y | L | V | | 3 |
| tr29 | L | L | M | D | C | S | G | S | I | | 51 |
| tr39 | G | I | A | G | G | L | A | L | L | | 500 |
| Is10 | I | L | Y | I | S | F | Y | F | I | | 4 |
| Is11 | Y | I | S | F | Y | F | I | L | V | | 6 |
| Is19 | G | I | Y | K | E | L | E | D | L | | 1801 |
| Is23 | H | I | F | D | G | D | N | E | I | | 1883 |
| cp36 | Y | L | K | T | I | Q | N | S | L | | 334 |
| cp37 | Y | L | Q | K | I | Q | N | S | L | | 334 |
| cp38 | Y | L | Q | K | I | K | N | S | L | | 334 |
| cp39 | Y | L | N | K | I | Q | N | S | L | | 334 |
these peptides being selected from three *Plasmodium falciparum* antigens, circumsporozoite protein (cp), thrombospondin-related anonymous protein (tr) and liver-stage antigen-1 (Is).

2. A peptide comprising at least two of the sequences listed in claim 1.

3. A peptide as claimed in claim 1 or claim 2 having an N-terminus or C-terminus carrying a lipid tail.

4. A peptide as claimed in any one of claims 1 to 3, comprising 8-100 amino acid residues.

5. A vaccine comprising at least one peptide according to any one of claims 1 to 4, for immunisation against malaria.

6. Oligonucleotides which code for the peptides claimed in any one of claims 1 to 4.

7. A vaccine comprising at least one oligonucleotide according to claim 6 for expression *in vivo* for immunization against malaria.

## Patentansprüche

1. Peptide, die entweder Epitope oder mögliche Epitope für HLA (humanes Leukozytenantigen) -A2-Klasse l-Moleküle sind, konservative Varianten davon und längere Peptide, die diese Sequenzen, welche Untereinheiten der angegebenen Antigene sind, enthalten:
| Bezeichnung | | Sequenz | | | | | | | | | Position |
|---|---|---|---|---|---|---|---|---|---|---|---|
| HLA-A2 | 1 | 2 | 3 | 4 | 5 | 6 | 7 | 8 | 9 | 10 | 1 |
| tr26 | H | L | G | N | V | K | Y | L | V | | 3 |
| tr29 | L | L | M | D | C | S | G | S | I | | 51 |
| tr39 | G | I | A | G | G | L | A | L | L | | 500 |
| Is10 | I | L | Y | I | S | F | Y | F | I | | 4 |
| Is11 | Y | I | S | F | Y | F | I | L | V | | 6 |
| Is19 | G | I | Y | K | E | L | E | D | L | | 1801 |
| Is23 | H | I | F | D | G | D | N | E | I | | 1883 |
| cp36 | Y | L | K | T | I | Q | N | S | L | | 334 |
| cp37 | Y | L | Q | K | I | Q | N | S | L | | 334 |
| cp38 | Y | L | Q | K | I | K | N | S | L | | 334 |
| cp39 | Y | L | N | K | I | Q | N | S | L | | 334 |
wobei diese Peptide ausgewählt sind aus drei *Plasmodium falciparum*-Antigenen, Circumsporozoitprotein (cp), thrombospondin-verwandtes anonymes Protein (tr) und Leber-Stadium-Antigen-1 (Is).

2. Peptid, welches wenigstens zwei der in Anspruch 1 aufgelisteten Sequenzen enthält.

3. Peptid nach Anspruch 1 oder Anspruch 2, welches einen N-Terminus oder C-Terminus hat, der einen Lipidschwanz trägt.

4. Peptid nach einem der Ansprüche 1 bis 3, welches 8-100 Aminosäurereste enthält.

5. Vaccin, welches wenigstens ein Peptid nach einem der Ansprüche 1 bis 4 enthält, zur Immunisierung gegen Malaria.

6. Oligonukleotide, welche die in einem der Ansprüche 1 bis 4 beanspruchten Peptide kodieren.

7. Vaccin, welches wenigstens ein Oligonukleotid nach Anspruch 6 zur Expression *in vivo* für eine Immunisierung gegen Malaria enthält.

## Revendications

1. Peptides, consistant en épitopes ou épitoges potentiels pour des molécules de HLA (antigène leucocytaire humain)-A2 de catégorie I, leurs variants conservateurs, et peptides plus longs contenant ces séquences qui sont des sous-unités des antigènes indiqués :
| Marqueur | Séquence | | | | | | | | | | Position |
|---|---|---|---|---|---|---|---|---|---|---|---|
| HLA-A2 | 1 | 2 | 3 | 4 | 5 | 6 | 7 | 8 | 9 | 10 | |
| tr26 | H | L | G | N | V | K | Y | L | v | | 3 |
| tr29 | L | L | M | D | C | S | G | S | I | | 51 |
| tr39 | G | I | A | G | G | L | A | L | L | | 500 |
| ls10 | I | L | Y | I | S | F | Y | F | I | | 4 |
| ls11 | Y | I | S | F | Y | F | I | L | V | | 5 |
| ls19 | G | I | Y | K | E | L | E | D | L | | 1801 |
| ls23 | E | r | F | D | G | D | N | E | I | | 1883 |
| cp3n | Y | L | K | T | I | Q | N | S | L | | 334 |
| cp37 | Y | L | Q | K | I | Q | N | S | L | | 334 |
| cp38 | Y | L | Q | K | I | K | N | S | L | | 334 |
| cp39 | Y | L | N | K | I | Q | N | S | L | | 334 |
ces peptides étant choisis parmi les antigènes de *Plasmodium falciparum*, la protéine de circumeporozoïte (cp), la protéine anonyme apparentée à la thrombospondine (tr) et l'antigène 1 de stade hépatique (ls).

2. Peptide comprenant au moins deux des séquences énumérées dans la revendication 1.

3. Peptide suivant la revendication 1 ou la revendication 2, ayant une extrémité N-terminale ou extrémité C-terminale portant une queue lipidique.

4. Peptide suivant l'une quelconque des revendications 1 à 3, comprenant 8 à 100 résidus d'amino-acides.

5. Vaccin comprenant au moins un peptide suivant l'une quelconque des revendications 1 à 4, pour l'immunisation contre le paludisme.

6. Oligonucléotides qui codent pour les peptides suivant l'une quelconque des revendications 1 à 4.

7. Vaccin comprenant au moins un oligonucléotide suivant la revendication 6 pour l'expression *in vivo* à des fins d'immunisation contre le paludisme.
